Europäisches Patentamt

European Patent Office

Office européen des brevets

(11) Publication number: **0 194 856 B1**

# EUROPEAN PATENT SPECIFICATION

(45) Date of publication of patent specification: **20.05.92** (51) Int. Cl.⁵: **A61F 9/00**, A61B 17/36

(21) Application number: **86301737.2**

(22) Date of filing: **11.03.86**

(54) **Surgical laser system.**

(30) Priority: **11.03.85 US 710431**
**18.10.85 US 788949**

(43) Date of publication of application:
**17.09.86 Bulletin 86/38**

(45) Publication of the grant of the patent:
**20.05.92 Bulletin 92/21**

(84) Designated Contracting States:
**AT BE CH DE FR GB IT LI LU NL SE**

(56) References cited:
**GB-A- 2 125 986**
**US-A- 4 316 467**
**US-A- 4 336 809**

(73) Proprietor: **ECLIPSE SURGICAL TECHNOL-
OGIES, INC.**
**Post Office Box 50875**
**Palo Alto, California 94303(US)**

(72) Inventor: **Mok, Walter Yiu Wa**
**639 Seale Avenue Palo Alto**
**California 94301(US)**

(74) Representative: **Wright, Hugh Ronald et al
Brookes & Martin 52/54 High Holborn
London WC1V 6SE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention pertains to the use of lasers in medicine and, more particularly, to the controllable firing of medical lasers when performing surgery.

Currently, medical and surgical laser output is guided visually by the operator. The eye, or an optical viewing device, is used to identify the treatment area and fire the laser. A major problem is that imperfect visualization of the treatment area leads to poor aim of the laser and consequently to damage of healthy tissue adjacent to the treatment area. When the laser device is accurately aimed, it is still difficult for the operator to know precisely the amount of laser energy to be delivered to destroy the treatment area without damaging the underlying tissue. Oversupply of laser energy may lead to an irreversible destruction of healthy tissue around the treatment site. This destruction can lead to side effects and complications from the procedure. Undersupply of laser energy may lead to inadequate destruction of the treatment area and a therapeutic failure. Furthermore, the problem is complicated because of the diversity of tissue types that are potential lesions.

U S patent 4,438,765 teaches the use of a laser surgical device wherein the controlling of the firing of the laser is by a motion detector to ensure that there is no movement of, say, the eyeball when the laser is fired for retinal fusion.

U S patent 4,316,467 teaches the use of a laser in removing naturally pigmented tissue from the skin. The firing of the laser is controlled by the color of the treatment area sensed by a photodetector. Both of these patents are basically concerned with the use of a laser in the surgical treatment of external body surfaces.

In order to enhance the visualization of the treatment area, there have been developed certain dyes which can selectively stain the diseased tissue. The difference in the optical property of the stained tissue and the unstained healthy tissue improves the visualization of the treatment area. U S patent 4,336,809 is typical of the teaching of a photoradiation method for tumor enhancement with hematoporphyrin dye, wherein the dyed lesion site is bathed with radiation of a particular wavelength to cause it to fluoresce.

In addition UK Patent 2125986 shows a laser arrangement for treatment of tumors. Operation of the apparatus relies upon the surgeon guiding and firing the apparatus until from his visual observation the tumor is removed. This may be suitable for a large tumor in an otherwise insensitive area, but clearly the time delay between the surgeon detecting the removal of the tumor and switching off the apparatus is liable to cause damage to healthy tissue.

When dealing with lesion sites within the body cavity, it is necessary to deliver the laser energy internally to the lesion site. U S patent 3,858,577 and U S patent 4,273,109 are typical of fiberoptic light delivery systems.

In spite of all of this existing technology, there is still not available a laser surgical system which is capable of performing laser surgery within the body cavity such that the laser effects are automatically monitored to control the output of the laser and to terminate its operation before there is a destruction of healthy tissue around the treatment site.

The present invention provides a surgical laser apparatus for operating on a treatment site comprising:

a fiber optical means having a proximal end and a distal end positionable in operative proximity to the treatment site,

a treatment laser source optically connected to the proximal end of said fiber optical means,

a diagnostic radiation source connected to the proximal end of said fiber optical means for irradiating the treatment site;

a responding radiation detector means connected to the proximal end of said fiber optical means characterised in that;

said radiation detector means generates a control signal when detecting a particular radiation emitted or reflected, in use, by the part of said treatment site to be treated in response to being irradiated by said diagnostic radiation source; and

means for controlling said treatment laser source to operate only when said radiation detector means emits said control signal.

The present invention also provides a surgical laser apparatus for operating on a treatment site comprising:

a fiber optical means having a proximal end and a distal end positionable in operative proximity to the treatment site;

a treatment laser source optically connected to the proximal end of said fiber optical means;

a diagnostic radiation source connected to the proximal end of said fiber optical means for irradiating the treatment site;

a responding radiation detector means connected to the proximal end of said fiber optical means;

whereby in use, the part of said treatment site to be treated provides a different response to the receipt of radiation from said diagnostic radiation source to the remainder of the treatment site;

characterised in that said radiation detector means is adapted to provide a control signal when detecting said different response of said part of said treatment site to be treated; and

means for controlling said treatment laser source to operate only when said radiation detector means emits said control signal.

The present invention also provides apparatus for destroying atheromatous plaque within an artery of a patient to whom has been administered a non-toxic atheroma-enhancing reagent which causes the plaque to have a characteristic optical property when illuminated with a given radiation,

## BRIEF DESCRIPTION OF DRAWINGS

Other features and advantages of the invention will be apparent from the following detailed description when read with the accompanying drawing in which:

Figure 1 is a block diagram of a laser system utilizing the invention;

Figure 2 is a schematic longitudinal section of the fiber optic cable of the system of Fig. 1;

Figure 3 is a cross-sectional view of said cable along the lines III-III of Fig. 2;

Figure 4 is a block diagram of a portion of the laser system of Figure 1 utilizing a single optical fiber; and

Figure 5 is a block diagram of a portion of the laser system of Figure 1 utilizing two optical fibers.

## DETAILED DESCRIPTION OF THE PREFERRED EMBODIMENTS OF THE INVENTION

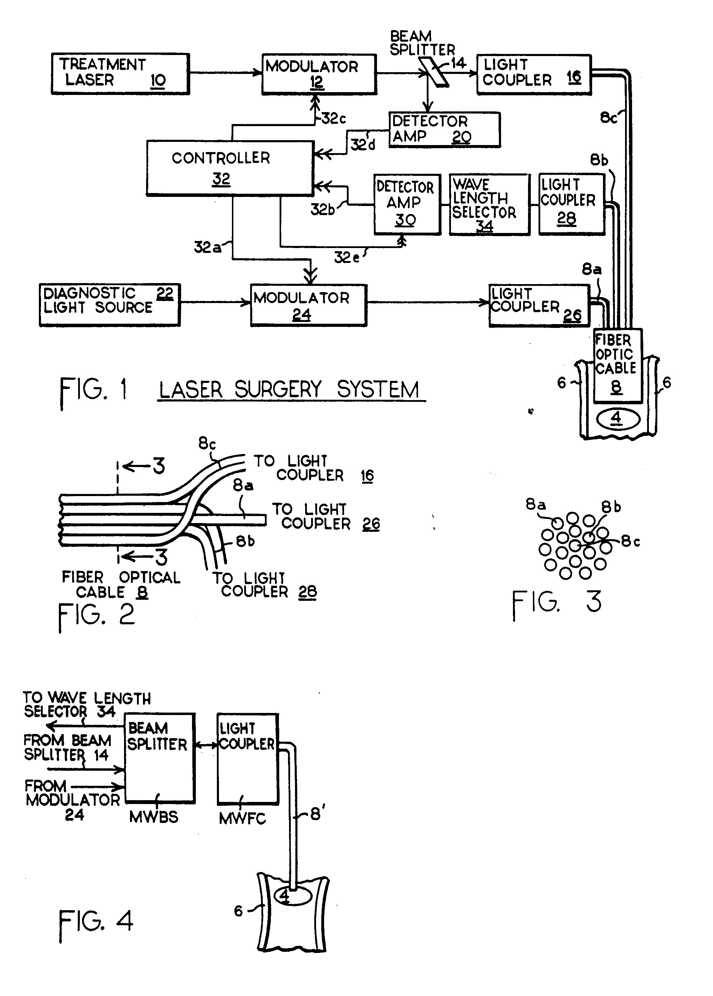

The invention will be described utilizing the example of the destruction of an atheromatous plaque 4 from the artery 6 of Fig. 1. Initially, the patient is administered a dose of a dye to enhance the contrast between the treatment site (plaque) and the healthy surrounding tissue. A typical dye is a tetracycline which has the property of fluorescing when radiated with an ultraviolet light. This dye has a special property of accumulating within the plaque relative to normal healthy tissue. Therefore, a predetermined time after the administration of the dye, the fiberoptic cable 8 is inserted into the artery with the distal end thereof opposite the treatment site. The optical cable 8 in a first embodiment (see also Figs. 2 and 3) includes a central optical fiber 8a coupled to the output of light coupler 26, an annular array of optical fibers 8b surrounding the central fiber 8a connected to the input of light coupler 28, and an outer annular array of cables of fibers 8c is coupled to the output of light coupler 16. Light coupler 26, of conventional design, receives light from the diagnostic light source 22 via the optical modulator 24. Diagnostic light source 22 for the present example would be a source of ultraviolet light. If other dyes were used, then appropriate light sources for those dyes would be selected. The light indicated by a single arrowhead line feeds the input of modulator 24 whose output is fed to the input of coupler 26. The modulator 24 can be of a conventional opto-accoustic modulator or an electromechanical shutter which passes or blocks the light in response to an electrical signal from controller 32 (note all electrical signal lines show double arrowheads). Thus, the presence or absence of a signal on line 32a from the controller 32 can close or open the light path between diagnostic light source 22 and light coupler 26.

The proximal end of array 8b feeds light into the light coupler 28 whose output is fed into the wavelength selector 34 which selects light corresponding to the predetermined characteristic wavelength to be detected. In turn, the selected light feeds detector-amplifier 30 whose output is fed via signal lead 32b to the controller 32. The detector-amplifier 30 can be the combination of, for example, a photodiode which drives a transistor amplifier, a photomultiplier, or in and of itself can be a phototransistor. Thus, whenever light is received from array 8b, a signal will be transmitted to controller 32. The detector/amplifier 30 is controlled by signals on line 32e from controller 32.

Treatment laser 10 will transmit light to modulator 12 which is controlled by signals on line 32c from controller 32. The controlled light from modulator 12 is fed to a conventional beam splitter 14 with a portion of light being deflected to detector/amplifier 20 and the remaining light passing to the input of light coupler 16. The output of light coupler 16 is fed to the proximal end of optical fiber array 8c. Beam splitter 14 also feeds part of the beam to detector/amplifier 20 which in turn feeds a signal on line 32d to controller 32 which provides feedback sensing of the laser output to ensure constancy of the amplitude of the laser output over time.

In operation, after the dye has been inserted and the cable 8 is in place, the diagnostic light source 22 passes light via modulator 24, coupler 26 and array 8a to the treatment site 4. The plaque in the treatment site will fluoresce and the fluorescence will be picked up by the array 8b and fed to the light coupler 28 and, thence, to the wavelength selector 34. The output from the wavelength selector 34, corresponding to the characteristic fluorescent emission of tetracycline, is fed to the detector/amplifier 30 which in response will emit a signal on line 32b to controller 32. The controller 32 in response thereto will send a signal on line 32c to open the modulator 12 to emit laser energy of a predetermined power and wavelength for a set time interval. Accordingly, a pulse of light from treatment laser 10 will be fed via the beam splitter, light coupler 16 and the array 8c to the treatment area 4. Because light reflected from the treatment area can be very great during the time of the laser pulse, controller 32 via line 32e feeds a signal to detector/amplifier 30 to turn off the detector for a predetermined time interval. This signal can also be fed to modulator 24 to prevent the radiation of ultraviolet light during the laser pulse. Controller 32 then switches signals on lines 32c and 32e at the predetermined timing delays such that the laser output is blocked and the fluorescent light can again be sensed from the treatment site. If the fluorescence is then detected indicating that plaque is still present, the detector 30 will send a signal to controller 32 which, again, switches the signals on the lines 32e and 32c, initiating another laser pulse. This sequence continues until no fluorescence is detected indicating that all plaque has been destroyed. At that time, no signal is fed to controller 32 and no further laser pulse is generated. In this way, using the probe-and-fire technique of the invention, the possibility of destroying healthy tissue is minimized. The controller 32 in its simplest form can dispense with the use of detector/amplifier 20 and can merely be a monostable device which is momentarily triggered on a pulse from line 32b and then reverts to its rest state. The paraphase output of this device can be connected via appropriate amplifiers to lines 32a, 32b and 32e

To facilitate the positioning of the laser catheter within narrow tortuous pathways a single flexible optical fiber 8' (or small diameter bundle) is used (see Fig.4) instead of the multibundle cable 8 of Fig. 1. More particularly, the light couplers 26, 28 and 16 connected to their associated bundles 8a, 8b and 8c are replaced by a single multiple-wavelength coupler MWFC which optically couples multi-wavelength beam splitter MWBS to single optical fiber 8'. Multiple-wavelength beam splitter MWBS receives laser light from beam splitter 14 (Fig. 1) along a given incident angle path and diagnostic light from modulator 24 (Fig. 1) along another given incident angle path and transmits such received light via a port along a common transmit-receive path to multiple-wavelength light coupler MWLC. Furthermore, radiation from the treatment site 4 is fed from multiple-wavelength coupler MWLC via the common transmit-receive path into the port of multiple-wavelength beam splitter MWBS. This light is emitted therefrom to wavelength fitter 34 via a further path having an angle different from the two given incident path angles. Because of the nature of the multiple-wavelength beam splitter MWBS it may be possible to delete fitter 34 and feed detector/amplifier 30 directly from the beam splitter.

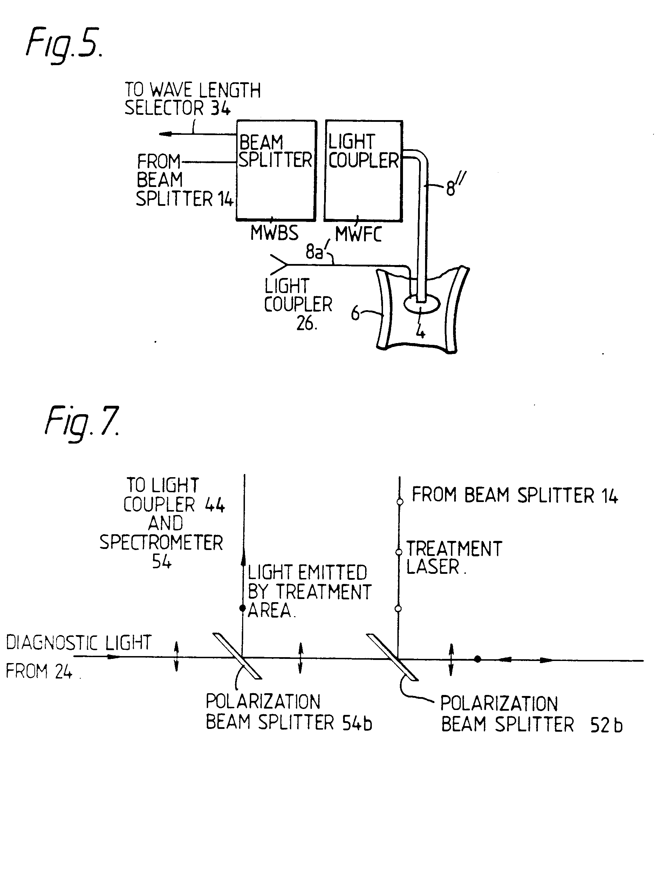

The so-modified system operates in the same manner as the system of Fig. 1. In Fig. 5 the fiber optical configuration is modified to a dual fiber configuration. This configuration may put less demands on the multiple-wavelength beam splitter MWBS and may permit more diagnostic light to reach the treatment site 4. In this embodiment a single fiber or bundle 8a' is connected to light coupler 26(Fig 1). The fiber 8" or narrow diameter cable is connected to multiple-wavelength light coupler MWFC which is optically-coupled via a common transmit-receive path to the ports of the multiple-wavelength beam splitter MWBS. Laser light is received along a given incident angle path from beam splitter 14 (Fig. 1) and fluorescent light from coupler MWFC is fed from multiple-wavelength beam splitter MWBS via an output optical path having a different angle to wavelength selector 34 (Fig. 1). As with the embodiment of Fig. 4 fitter 34 may be omitted.

Operation of the system utilizing the embodiment of Fig. 5 is the same as the other embodiments.

In addition to tetracycline it has been found that the dye Nile Red (9-di-ethylamino-5h-benzo($\alpha$) phenoxazine-5-one) shows excellent results with plaque.

While only a limited number of embodiments of the invention has been shown and described in detail, there will now be obvious to those skilled in the art many modifications and variations satisfying many or all of the objects and features of the invention without departing from the spirit thereof. For example, while only the treatment of plaque has been described the invention can be used as a treatment of other diseases such as tumors (cancer), stones in urinary tract and gall bladder as well as prostate obstructions. In addition, depending on the nature of the treatment site, the appropriate dye is selected to enhance the contrast between normal tissue and malignant tissue. When the treatment site is a tumor, one can successfully use hematoporphyrin or its derivatives. In some cases, inherent differences in optical properties between the treatment site and the surrounding healthy tissue may eliminate the need for the dye. Again, depending on the treatment site and the dyes involved, the diagnostic light can be ultraviolet, infra red, white light, etc. Furthermore, again depending on the treatment site, the laser source can take many forms such as argon, Nd-yag, carbon dioxide, tunable dye, and excimer lasers with pulse or continuous output. The choice of the diagnostic light source is predicated on the optical characteristics of the dye and/or the treatment site. However, the choice of the coherent light source for the treatment laser does not have to match the absorption peak of the dye. The treatment laser can be any wavelength that destroys the diseased treatment site. Normally, there is a risk that this light will also destroy healthy tissue. However, the possibility does not exist since once the diseased treatment site is removed, the means for triggering the laser pulse is also removed.

The fiberoptic cable can be coupled with catheter designs which include, but are not limited to, such features as endoscopy, balloon devices, steerable guiding systems, multiple lumens for infusion and suctioning, ultrasonic guidance, monitoring or ablation, pressure and temperature monitoring and catheter centering devices.

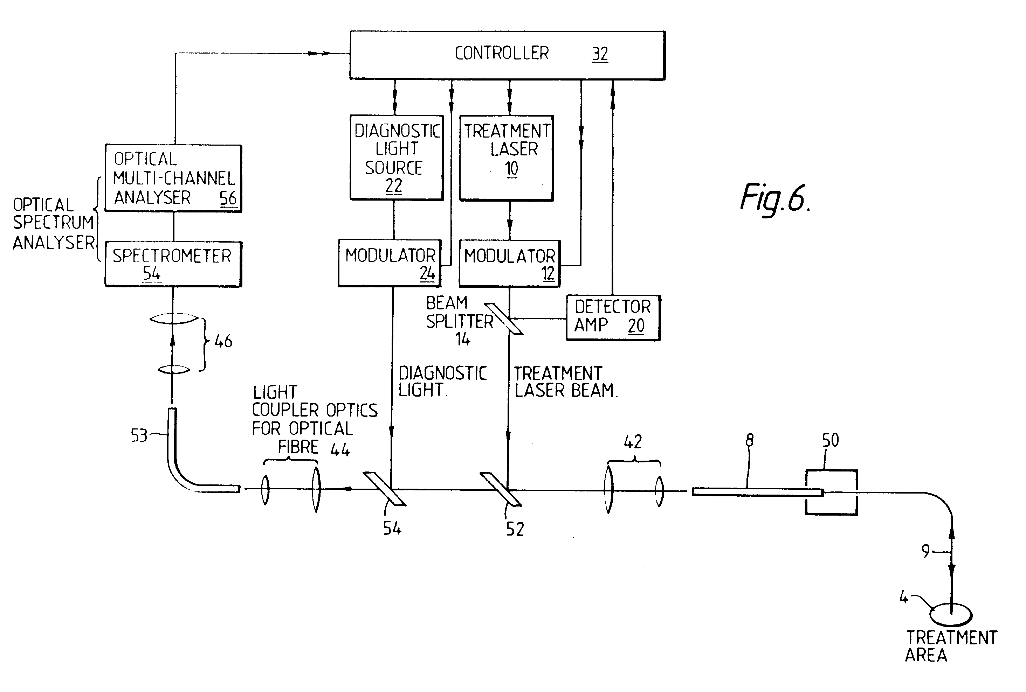

Further embodiments of the invention will be described with reference to Figures 6 to 8.

The "Probe and Fire" laser system of Figure 6 utilizes the optical characteristic properties of the treatment area as the input to a control system which controls the firing of the treatment laser.

The "Probe and Fire" laser system consists of the following major components - (1) a diagnostic light source to illuminate the treatment area, (2) various light delivery fibers and optics, (3) optical multichannel analyzer and spectrometer to capture the emission spectrum of the treatment area, (4) a treatment laser and a (5) computer control system capable of performing signal processing of the emission spectrum from the treatment area, controlling the optical characteristics of the light diagnostic light source and treatment laser such as wavelength, pulse width, amplitude and firing duration.

The instrument is operated in the following manner. The controller 32 first actives the diagnostic light source 22 and the treatment laser 10 in a "ready" mode. The illumination duration intensity and wavelength are controlled by modulator 24. The diagnostic light is reflected by beam splitter 54 which has high reflectivity at diagnostic light wavelengths and transmits light at other wavelengths. Beam splitter 52 is transparent to the diagnostic light which is then focused on the optical fiber 8 through the optical fiber coupler 42. The optical fiber is mated with a catheter 9 - through a catheter coupler 50. The treatment area is illuminated by the diagnostic light and its emission spectrum is collected by optical fiber 9. The light retraces the optical path of the diagnostic light. Beam splitter 52 is transparent to the characteristic light and the same is true for beam splitter 54. The characteristic spectral light goes through Beam Splitter 54 and is coupled into optical fiber 58 by the optical fiber coupler 44. The light emerging from optical fiber 58 is focused into the entrance slit of a spectrometer 55 by a coupling optic 46. The full emission spectrum is recorded by an optical multichannel analyser 56. The spectrum is then processed by the computer in the controller 32. Certain characteristics (eg: wavelength) of the spectrum are identified as a means to discriminate the desired treatment area and to collect diagnostic information from the treatment area. After the desired treatment area is identified and the condition of the area is known the command is given by the controller to fire the treatment laser 10 at a given wavelength, intensity, and duration. Intensity and wavelength are controlled by the modulator 12. Beam splitter 14 diverges a small fraction of the light from the treatment laser into detector amplifier 20 which is fed back to the controller as a means to monitor the laser output. The treatment laser light is reflected into the fiber optics delivery system by beam splitter 52 which has high reflectivity at treatment laser wavelength and transmits light at other wavelengths. The treatment laser light is coupled into optical fiber 8 by optical coupler 42 and the light is delivered into the catheter by coupler 50. It reaches the treatment area 4 through catheter 9.

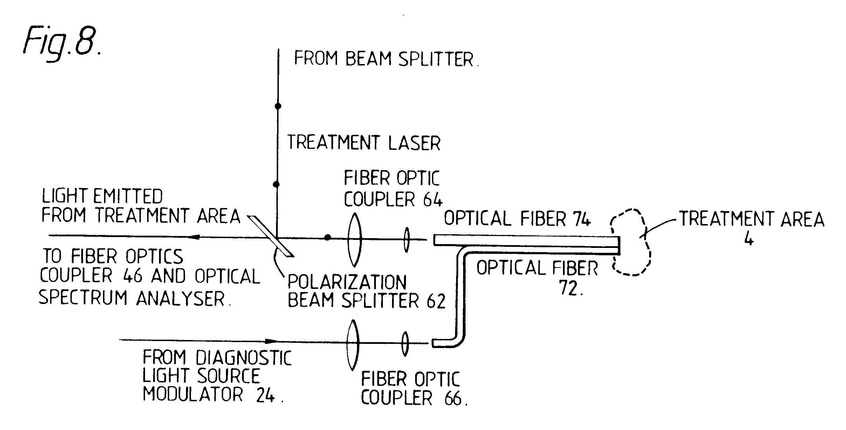

After the firing procedure of the treatment laser is completed, the treatment area is examined by the diagnostic light again. The emission spectrum from the treated area will determine whether repeated firing is required or not. Referring to Figure 7. A more detailed embodiment of the system is for variable wavelength application. For multiwavelength applications, the beam splitters 54 and 52 are replaced with polarization beam splitters 54b, 52b which reflect light with polarization perpendicular to the plan of incidence and transmits light parallel to the plan of incidence. The treatment laser will be "P" polarized with respect to the beam splitter 52 and the diagnostic light source will be "S" polarized with respect to beam splitter 54. The diagnostic light will pass through both beam splitters 54b and 52b and be focused on optical fiber 8 by optical coupler 42.

The emission spectrum returning from the treatment area is usually randomly polarized, half of it goes through beam splitter 52b and half of the remaining is reflected into the spectrum analyzer module which is composed of a spectrometer, optical multichannel analyzer and computer.

The diagnostic light coming out of the light source and modulator is polarized and with its polarization vector perpendicular to the plane of incidence. It passes through the polarizing beam splitter 54b and polarizing beam splitter 52b and is coupled into optical fiber 8 by fiber optic coupler 42. The light emitted by the treatment area after being excited by the diagnostc light, is transmitted back to the instrument by optic fiber 9, catheter coupler 50 and optic fiber 8 as in figure 1, and coupler 42 as shown in figure 6. It then transmitts through polarization beam splitter 52b partially and is reflected into the light coupler 44 partially.

Both embodiments of the laser system instruments can be used, if desired for fluorescence detection only. The sensitivity of the instrument can thus be enhanced by removing the beam splitter 52 which couples the treatment laser beam into the system when the system is used only as fluorescence detection device.

The arrangement of Figure 7 allows the use of a broader range of operating wavelengths for the diagnostic light and the treatment of light.

Another embodiment is a dual channel laser surgical and fluorescent detection system. The diagnostic light and the treatment laser light are transmitted through two different fibers as is shown in figure 8. This allows diagnostic light of variable wavelength to be coupled into the fiber. The diagnostic light coming out of the modulator 24 is coupled into optic fiber 72 with fiber optic coupler 66. It illuminates the treatment area 4. Light emitted by the treatment area 4 is collected by optic fiber 74 and directed into the optical spectrum analyser through the fiber optic coupler 64 and polarizing beam splitter 62. After the signal processing procedure, if the treatment laser is given a command to fire, the treatment laser will give out polarized laser light with the polarization vector perpendicular to the incidence plane of the polarizing beam splitter and is reflected into the fiber optic coupler and it is then transmitted through fiber 74 into the treatment area. To enhance the sensitivity of the system, the fiber tip of 72 can be a convex lens such that it focuses the treatment light at a distance away from the fiber exit surface. This system can also be efficiently used as a fluorescence detection system.

**Claims**

1. A surgical laser apparatus for operating on a treatment site comprising:
   a fiber optical means (8) having a proximal end and a distal end positionable in operative proximity to the treatment site (4),
   a treatment laser source (10) optically connected to the proximal end of said fiber optical means (8),
   a diagnostic radiation source (22) connected to the proximal end of said fiber optical means (8) for irradiating the treatment site (4);
   a responding radiation detector means (30) connected to the proximal end of said fiber optical means (8) characterised in that;
   said radiation detector means (30) generates a control signal when detecting a particular radiation emitted or reflected, in use, by the part of said treatment site to be treated in response to being irradiated by said diagnostic radiation source (22); and
   means (32) for controlling said treatment laser source to operate only when said radiation detector means (30) emits said control signal.

2. The apparatus as claimed in claim 1 characterised in that said fiber optical means comprises a multiple-wavelength beam splitter (MWBS), a fiber optical device (8') and a multiple-wavelength optical coupler (MWFC) optically coupling said multiple-wavelength beam splitter (MWBS) to said fiber optical device (8').

3. The apparatus as claimed in claim 1 characterised in that said fiber optical means (8) comprises first and second fiber optical devices (8",8a'), a multiple-wavelength beam splitter (MWBS) optically coupled to said treatment laser source (10) and to said responding radiation detector (30), a multiple-wavelength optical coupler (MWFC) optically coupling said diagnostic radiation source (22) to said second fiber optical device (8a').

4. The apparatus as claimed in claim 3 characterised in that at least one of said fiber optical devices (8) is a single optical fiber (8).

5. The apparatus as claimed in claim 3 characterised in that at least one of said fiber optical devices (8) is a bundle of optical fibers (8).

6. The apparatus as claimed in claim 1 characterised in that said fiber optical means (8) is a fiber optical device having a central fiber (8a), optical diagnostic means with a proximal end coupled to said diagnostic radiation source (22), a receiving fiber optical array means (8b) annularly disposed about said diagnostic means with a proximal end coupled to said detector (30), and a treatment fiber optical array (8c) also annularly disposed about said diagnostic means with one end coupled to said treatment laser source (10).

7. The apparatus as claimed in claim 1 characterised in that the radiation detector means is arranged to detect radiation of a particular wavelength or wavelengths only.

8. The apparatus as claimed in any of claims 1 to 7 characterisd in that the radiation from the part to be treated is provided by fluorescence of the part to be treated emitted in response to receipt of diagnostic radiation from the diagnostic radiation source (22).

9. The apparatus as claimed in any of claims 2 to 8 characterised in that the controller means (32) includes means to control said treatment laser source (10) to provide a series of bursts of laser radiation.

10. The apparatus as claimed in any of claims 1 to 9 charcterised in that the distal end of the fiber optical means (8) is of a form suitable for insertion into the arteries of a patient.

11. A surgical laser apparatus for operating on a treatment site comprising:
a fiber optical means (8) having a proximal end and a distal end positionable in operative proximity to the treatment site (4);
a treatment laser source (10) optically connected to the proximal end of said fiber optical means (8);
a diagnostic radiation source (22) connected to the proximal end of said fiber optical means (8) for irradiating the treatment site (4);
a responding radiation detector means (30) connected to the proximal end of said fiber optical means (8);
whereby in use, the part of said treatment site to be treated provides a different response to the receipt of radiation from said diagnostic radiation source to the remainder of the treatment site characterised in that;
said radiation detector means (30) is adapted to provide a control signal when detecting said different response of said part of said treatment site to be treated; and
means (32) for controlling said treatment laser source (10) to operate only when said radiation detector means (30) emits said control signal.

**Revendications**

1. Appareil chirurgical à laser pour opérer sur un site de traitement, comprenant :
   - un moyen à fibres optiques (8) ayant une extrémité proche et une extrémité éloignée pouvant être positionnée à proximité opératoire du site de traitement (4);
   - une source laser de traitement (10) reliée optiquement à l'extrémité proche dudit moyen à fibres optiques (8);
   - une source de rayonnement de diagnostic (22) reliée à l'extrémité proche dudit moyen à fibres optiques (8) pour irradier le site de traitement (4);
   - un moyen répondeur, détecteur de radiation (30) relié à l'extrémité proche dudit moyen à fibres optiques (8),
   **caractérisé** en ce que :
   - ledit moyen détecteur de rayonnement (30) produit un signal de commande quand il détecte un rayonnement particulier émis ou réfléchi, lors de l'utilisation, par la partie dudit site de traitement à traiter, lorsqu'elle est irradiée par ladite source (22) de rayonnement de diagnostic; et
   - un moyen (32) pour commander ladite source laser de traitement pour qu'elle opère seulement quand ledit moyen détecteur de rayonnement (30) émet ledit signal de commande.

2. Appareil selon la revendication 1, caractérisé en ce que ledit moyen à fibres optiques comprend un diviseur de faisceau à longueurs d'ondes multiples (MWBS), un dispositif à fibre optique (8') et un coupleur optique à longueur d'ondes multiples (MWFC) couplant optiquement ledit diviseur de faisceau à longueurs d'ondes multiples (MWBS) avec ledit dispositif à fibre optique (8').

3. Appareil selon la revendication 1, caractérisé en ce que ledit moyen à fibres optiques (8) comprend un premier et un deuxième dispositifs à fibres optiques (8'', 8a'), un diviseur de faisceau à longueurs d'ondes multiples (MWBS) couplé optiquement à ladite source laser de traitement (10) et audit détecteur répondant au rayonnement (30), et un coupleur optique à longueurs d'ondes multiples (MWFC) couplant optiquement ladite source de rayonnement de diagnostic (22) audit deuxième dispositif à fibre optique (8a').

4. Appareil selon la revendication 3, caractérisé en ce qu'un au moins desdits dispositifs à fibres optiques (8) est une monofibre optique (8).

5. Appareil selon la revendication 3, caractérisé en ce qu'un au moins desdits dispositifs à fibres optiques (8) est un faisceau de fibres optiques (8).

6. Appareil selon la revendication 1, caractérisé en ce que ledit moyen à fibres optiques (8) est un dispositif à fibres optiques ayant une fibre centrale (8a), un moyen optique de diagnostic avec une extrémité proche couplée à ladite source de rayonnement de diagnostic (22), un moyen récepteur à fibres optiques groupées (8b) disposées en anneau autour dudit moyen de diagnostic, avec une extrémité proche couplée audit détecteur (30), et un groupe de fibres optiques de traitement (8c) également disposées en anneau autour dudit moyen de diagnostic, avec une extrémité couplée à ladit source laser de traitement (10).

7. Appareil selon la revendication 1, caractérisé en ce que le moyen détecteur de rayonnement est agencé pour détecter seulement le rayonnement d'une ou plusieurs longueurs d'ondes particulières.

8. Appareil selon l'une quelconque des revendications 1 à 7, caractérisé en ce que le rayonnement de la partie à traiter est fourni par fluorescence de la partie à traiter, émise en réponse à la réception du rayonnement de diagnostic venant de la source de rayonnement de diagnostic (22).

9. Appareil selon l'une quelconque des revendications 2 à 8, caractérisé en ce que le moyen de commande (32) comprend des moyens pour commander ladite source laser de traitement (10) pour fournir une série de rafales de rayonnement laser.

10. Appareil selon l'une quelconque des revendications 1 à 9, caractérisé en ce que l'extrémité éloignée du moyen à fibres optiques (8) a une forme convenant à être insérée dans les artères d'un patient.

11. Appareil chirurgical à laser pour opérer sur un site de traitement comprenant :
    - un moyen à fibres optiques (8) ayant une extrémité proche et une extrémité éloignée pouvant être positionnée à proximité opératoire du site de traitement (4);
    - une source laser de traitement (10) reliée optiquement à l'extrémité proche dudit moyen à fibres optiques (8);
    - une source de rayonnement de diagnostic (22) reliée à l'extrémité proche dudit moyen à fibres optiques (8) pour irradier le site de traitement (4);
    - un moyen répondeur, détecteur de rayonnement (30) relié à l'extrémité proche dudit moyen à fibres optiques (8);
    au moyen desquels la partie dudit site de traitement devant être traitée fournit une réponse différente à la réception du rayonnement venant de ladite source de rayonnement de diagnostic, par rapport au reste du site de traitement;
    **caractérisé** en ce que :
    - ledit moyen détecteur de rayonnement (30) est adapté pour fournir un signal de commande quand il détecte ladite réponse différente de ladite partie dudit site de traitement devant être traitée; et

    - un moyen (32) pour commander ladite source laser de traitement (10) pour qu'elle fonctionne seulement quand ledit moyen détecteur de rayonnement (30) émet ledit signal de commande.

**Patentansprüche**

1. Chirurgische Laser-Vorrichtung zur Anwendung an einem Behandlungsort, umfassend:
   eine faser-optische Vorrichtung (8) mit einem proximalen und einem distalen Ende, zum Positionieren in operativer Nähe beim Behandlungsort (4);
   eine Laser-Behandlungs-Quelle (10), die optisch mit dem proximalen Ende der faser-optischen Vorrichtung (8) verbunden ist;
   eine diagnostische Strahlenquelle (22), die mit dem proximalen Ende der faser-optischen Vorrichtung (8) zum Bestrahlen des Behandlungsortes (4) verbunden ist;
   einen darauf ansprechenden Strahlungsdetektor (30), der mit dem proximalen Ende der faser-optischen Vorrichtung (8) verbunden ist, dadurch gekennzeichnet, daß der Strahlendetektor (30) ein Kontrollsignal beim Gebrauch dann abgibt, wenn er eine bestimmte Strahlung erfaßt, die emittiert oder reflektiert wird, und zwar von dem zu behandelnden Teil des Behandlungsortes in Abhängigkeit von dessen Bestrahlung mit der diagnostischen Bestrahlungsquelle (22); und
   daß Mittel (22) zum Kontrollieren der Laser-Behandlungs-Quelle vorgesehen sind, und daß diese nur dann arbeiten, wenn der Strahlungsdetektor (30) das genannte Kontrollsignal abgibt.

2. Vorrichtung nach Anspruch 1, dadurch gekennzeichnet, daß das faser-optische Gerät einen Multipel-Wellenlängen-Strahlteiler (MWBS) umfaßt, ein faser-optisches Gerät (8') sowie einen optischen Multipel-Wellenlängen-Koppler (MWFC), der den Multipel-Wellenlängen-Strahlteiler (MWBS) mit dem optischen Gerät (8') verbindet.

3. Vorrichtung nach Anspruch 1, dadurch gekennzeichnet, daß das faser-optische Gerät (8) ein erstes und ein zweites faseroptisches Gerät (8'', 8a') umfaßt, einen Multipel-Wellenlängen-Stahlteiler (MWBS), der mit der Laser-Behandlungs-Quelle (18) und dem ansprechenden Strahlendetektor (30) zusammengeschaltet ist, sowie einen optischen Multipel-Wellenlängen-Koppler (MWFC), der die diagnostische Strahlungsquelle (22) mit dem zweiten faser-optischen Gerät (8a') optisch zusammenschaltet.

4. Vorrichtung nach Anspruch 3, dadurch gekennzeichnet, daß wenigstens eines der faser-optischen Geräte (8) eine einzelne optische Faser (8) ist.

5. Vorrichtung nach Anspruch 3, dadurch gekennzeichnet, daß wenigstens eines der genannten faser-optischen Geräte (8) ein Bündel optischer Fasern (8) ist.

6. Vorrichtung nach Anspruch 1, dadurch gekennzeichnet, daß das faser-optische Gerät (8), ein Gerät ist, das eine zentrale Faser (8a) aufweist, ein optisches Diagnostiziergerät, dessen proximales Ende an die diagnostische Strahlungsquelle (22) anschließt, eine empfangende faser-optische Anordnung (8b), die um das Diagnostiziergerät herum ringförmig angeordnet ist, wobei ein proximales Ende an den Detektor (30) angeschlossen ist, sowie eine faser-optische Behandlungsanordnung (8c), die ebenfalls ringförmig um das Diagnostiziergerät herum angeordnet ist, wobei ein Ende an die Laser-Behandlungs-Quelle (10) angeschlossen ist.

7. Vorrichtung nach Anspruch 1, dadurch gekennzeichnet, daß der Strahlungsdetektor derart angeordnet ist, daß er allein die Strahlung einer bestimmten Wellenlänge oder bestimmter Wellenlängen erfaßt.

8. Vorrichtung nach einem der Ansprüche 1 bis 7, dadurch gekennzeichnet, daß die Strahlung von dem zu behandelnden Teil durch Fluoreszenz des zu behandelnden Teils erzeugt wird, emittiert in Abhängigkeit von dem Empfang diagnostischer Strahlen aus der diagnostischen Strahlungsquelle (22).

9. Vorrichtung nach einem der Ansprüche 2 bis 8, dadurch gekennzeichnet, daß der Regler (32) Mittel zum Regeln der Laser-Behandlungs-Quelle (10) umfaßt, um eine Reihe von Bursts der Laserstrahlung zu erzeugen.

10. Vorrichtung nach einem der Ansprüche 1 bis 9, dadurch gekennzeichnet, daß das distale Ende des faser-optischen Gerätes (8) eine Gestalt aufweist, die geeignet zum Einführen in die Arterie eines Patienten ist.

11. Chirurgische Laser-Vorrichtung zur Anwendung an einem Behandlungsort umfassend:
ein faser-optisches Gerät (8), das ein proximales und ein distales Ende aufweist, und das in operativer Nähe zum Behandlungsort (4) positionierbar ist;
eine Laser-Behandlungs-Quelle (10), die optisch mit dem proximalen Ende des faser-optischen Gerätes (8) verbunden ist;
eine diagnostische Strahlungsquelle (22), die mit dem proximalen Ende des faser-optischen Gerätes (8) zum Bestrahlen des Behandlungsortes (4) verbunden ist;
ein ansprechender Strahlungsdetektor (30), der mit dem proximalen Ende des faser-optischen Gerätes (8) verbunden ist;
wobei das zu behandelnde Teil des Behandlungsortes beim Empfang von Strahlung von der diagnostischen Strahlungsquelle ein anderes Signal liefert, als der übrige Behandlungsort, dadurch gekennzeichnet,
daß der Strahlungsdetektor (30) dazu vorgesehen ist, daß er Regelsignal dann abgibt, wenn er von dem zu behandelnden Teil des Behandlungsortes eines der genannten unterschiedlichen Signale erhält; und
daß Mittel (32) zum Regeln der Laser-Behandlungs-Quelle (10) vorgesehen sind, die nur dann arbeiten, wenn der Strahlungsdetektor (30) das genannte Regelsignal emittiert.

FIG. 1  LASER SURGERY SYSTEM

FIG. 2

FIG. 3

FIG. 4

# Fig.5.

TO WAVE LENGTH
SELECTOR 34

FROM
BEAM
SPLITTER 14

| BEAM SPLITTER | LIGHT COUPLER |

8''

MWBS    MWFC

8a

LIGHT
COUPLER
26.

6

4

# Fig.7.

TO LIGHT
COUPLER 44
AND
SPECTROMETER
54

FROM BEAM SPLITTER 14

LIGHT EMITTED
BY TREATMENT
AREA.

TREATMENT
LASER.

DIAGNOSTIC LIGHT

FROM 24.

POLARIZATION
BEAM SPLITTER 54b

POLARIZATION
BEAM SPLITTER 52b

Fig.6.

CONTROLLER  32

OPTICAL
SPECTRUM
ANALYSER {

OPTICAL
MULTI-CHANNEL
ANALYSER  56

SPECTROMETER
54

DIAGNOSTIC
LIGHT
SOURCE
22

TREATMENT
LASER
10

MODULATOR
24

MODULATOR
12

BEAM
SPLITTER
14

DETECTOR
AMP  20

46

53

LIGHT
COUPLER OPTICS
FOR OPTICAL
FIBRE  44

DIAGNOSTIC
LIGHT.

TREATMENT
LASER BEAM.

42

8

50

54

52

9

4

TREATMENT
AREA

EP 0 194 856 B1

Fig.8.

FROM BEAM SPLITTER.

TREATMENT LASER

FIBER OPTIC COUPLER 64

LIGHT EMITTED FROM TREATMENT AREA

POLARIZATION BEAM SPLITTER 62

TO FIBER OPTICS COUPLER 46 AND OPTICAL SPECTRUM ANALYSER.

FROM DIAGNOSTIC LIGHT SOURCE MODULATOR 24.

FIBER OPTIC COUPLER 66.

OPTICAL FIBER 74

OPTICAL FIBER 72.

TREATMENT AREA 4